(19) Europäisches Patentamt
European Patent Office
Office européen des brevets

(11) Publication number: **0 477 999 A1**

## EUROPEAN PATENT APPLICATION

(21) Application number: **91118723.5**

(22) Date of filing: **10.12.85**

(51) Int. Cl.⁵: **G01N 33/72**, //C12Q1/28

This application was filed on 04 - 11 - 1991 as a divisional application to the application mentioned under INID code 60.

(30) Priority: **11.12.84 US 680357**

(43) Date of publication of application:
**01.04.92 Bulletin 92/14**

(60) Publication number of the earlier application in accordance with Art.76 EPC: **0 204 833**

(84) Designated Contracting States:
**AT BE CH DE FR GB IT LI LU NL SE**

(71) Applicant: **LAWRENCE, Paul J.**
**2082 Abbey Lane**
**Campbell, CA 95008(US)**

(72) Inventor: **LAWRENCE, Paul J.**
**2082 Abbey Lane**
**Campbell, CA 95008(US)**

(74) Representative: **Goldin, Douglas Michael et al**
**J.A. KEMP & CO. 14, South Square Gray's Inn**
**London WC1R 5EU(GB)**

(54) **Fecal occult blood test reagent and methods.**

(57) Fecal occult blood tests of enhanced sensitivity, specificity, and readability are provided by employing (1) a developer or complete reagent solution that uses as a solvent system a solvent comprising at least about 50% by volume of a solvent for iron protoporphyrins such as dimethyl sulfoxide and/or (2) a multi-chromagen comprised of a mixture of guaiac and ABTS. Test results may be further improved by incorporating hemoprotein solubilizing agents, plant peroxidase inhibitors, iron/copper chelating agents, and buffers in the developer/reagent.

EP 0 477 999 A1

Technical Field

This invention is in the field of fecal occult blood tests (FOBT). More particularly it relates to improved FOBT that provide a reduced incidence of false results and/or greater sensitivity and specificity and/or are easier to perform.

Background Art

FOBT are commonly used clinically to detect occult blood loss from gastrointestinal (GI) lesions. For example, carcinoma of the colon and rectum is the most serious cancer in the U.S. and second only to lung cancer in causing death--approximately 100.000 new cases and 50.000 deaths annually. Because colorectal cancer is slowly progressive with a long asymptomatic period, it provides an ideal opportunity for early detection and successful therapy. Thus, FOBT are a rational attempt at early diagnosis because the colorectal lesions frequently bleed, and routine noninvasive testing is possible. Similarly, hospitals and physicians very often utilize FOBT to detect or monitor GI lesions resulting from disease, injury, surgery, and other causes.

Early FOBT involved shipping entire 24-48 hour fecal collections in paint cans to central laboratories for testing with an acidified guaiac solution and hydrogen peroxide. Guaiac is a complex plant extract containing the leuco dye, alpha guaiaconic acid. Leuco dyes are oxidized by hydroperoxides in the presence of catalyst to form a blue color:

$$\textsf{Reduced Leuco dye} \xrightarrow[\textsf{catalyst}]{\textsf{hydroperoxide}} \textsf{Oxidized Leuco dye}$$
$$\textsf{(colorless)} \qquad\qquad\qquad\qquad \textsf{(blue)}$$

Because hemoglobin is an efficient catalyst (pseudoperoxidase), feces may be tested for occult blood using a leuco dye/hydroperoxide reagent. Nonetheless, the procedure remained very poorly utilized because of the disagreeable nature of the test and physicians were largely denied this very useful information.

U.S. Patent No. 3,996,006 describes a FOBT technique that popularized the guaiac-based test for occult blood in feces. It employs a slide having a sheet of guaiac-impregnated paper between a front panel and a rear panel with openings in the panels and pivotal flaps to cover the openings. A fecal specimen is placed on the paper through the opening in the front panel and that panel is closed. The rear panel is then opened and a hydrogen peroxide developer is placed on the paper via the opening in the rear panel. If blood is present in the specimen, the paper will turn blue. A commercial embodiment of this test, called the HEMOCCULT® test, is widely used in hospitals and physicians' offices. Despite the widespread popularity of the HEMOCCULT® test, recent studies have pointed out serious limitations in its sensitivity and specificity. Applicant believes that the sensitivity limitation is due partly to (1) the fact that hemoglobin in many specimens is degraded to hemoproteins that exhibit little or no peroxidative activity, (2) degradation of peroxidatively active hemoproteins by the hydroperoxide reagent used in the test and (3) the relative insolubility of the degraded products (i.e., iron protoporphyrins such as heme and hemin) in the reagents used in the test. Sensitivity limitations, of course, may cause false negative results. The specificity limitation is probably due to the response of the test to plant peroxidases and/or iron or copper in the specimens or the environment in which the test is run. Specificity limitations lead to false positive results.

U.S. Patent No. 4,333,734 describes a variation in the guaiac-based FOBT that is intended to reduce the incidence of false positive results due to the presence of plant peroxidases in the specimen. It includes a peroxidase denaturing agent such as urea or guanidine hydrochloride together with a metal chelating agent to sequester calcium and magnesium ions that are essential to peroxidase activity. The denaturant and the chelating agent are formulated with the guaiac.

U.S. Patent No. 4,071.317 relates to using polar solvents such as dimethyl sulfoxide (DMSO) and dimethyl formamide (DMF) to stabilize mixtures of organic hydroperoxides and leuco dyes that are used in FOBT. The solvent is formulated in minor proportions with the hydroperoxide and leuco dye. This solution is applied to a solid matrix and the matrix is dried prior to use in testing.

Several references indicate that monomeric species of iron protoporphyrins exhibit greater peroxidase activity than dimeric or aggregated species. Biochem J (1970) 117: 741-744; Biochem J (1973) 135: 353-359; Biochem J (1976) 153: 279-295; and Biochemistry (1974) 13: 4279-4284. Biochem J (1979) 179: 291-289 indicates that hemin occurs in its monomeric form in mixtures of DMSO and water that contain in excess of about 35% (v/v) DMSO.

Biochem J (1960) 108: 131-136 discusses the solubility of nitrogenous ligand-alkaline hematin complexes. Biochimica et Biophysica Acta (1977) 498: 205-214 describes the use of various water-soluble polymers such as polyethylene oxide, polyvinyl alcohol, polyvinyl pyrrolidone, and polystyrene sulfonate to dissolve aggregates of ferroheme and protoporphyrin in alkaline aqueous media.

An object of the present invention is to reduce the incidence of incorrect results (both false positive and false negative) in leuco dye-based FOBT. This is achieved by applying the hydroperoxide or both the hydroperoxide and leuco dye to the specimen in a solution that uses a solvent system that dissolves iron protoporphyrins. Another object is to provide a chromagen that improves test readability and a complete FOBT reagent containing that chromagen.

Disclosure of the Invention

One aspect of the invention is a chromagen for use in a fecal occult blood test characterized in that the chromagen is a mixture of guaiac and 2,2'-azino-di-(3-ethylbenzyl)azoline sulfonic acid sulfonic acid (ABTS).

Another aspect of the invention is a fecal occult blood test method comprising contacting a fecal specimen on a solid test matrix with a leuco dye and a hydroperoxide characterized in that the hydroperoxide or the hydroperoxide and the leuco dye are in solution in a solvent comprising at least 50% by volume of a solvent for iron protoporphyrins whereby the iron protoporphyrins in the specimen are dissolved.

Still another aspect of the invention is a fecal occult blood test method comprising contacting a fecal specimen on a solid test matrix with a leuco dye and a hydroperoxide characterized in that the leuco dye comprises a mixture of guaiac and ABTS.

Modes for Carrying Out the Invention

As used herein the term "hemoprotein" is intended to include hemoglobin and derivatives of hemoglobin such as heme and hemin that have the ability (particularly in their monomeric form) to catalyze the transfer of oxygen from a hydroperoxide to a leuco dye to cause the leuco dye to be oxidized and thereby produce a detectable response. Such ability is sometimes referred to herein as "peroxidative activity".

As used herein the term "leuco dye" is not intended to be limited to a particular chemical species or genus but is intended to encompass indicators that produce a detectable response, typically a color change that is visible to the naked eye, when oxidized in the presence of a hemoprotein. Examples of leuco dyes are guaiac, benzidine, o-tolidine, cresol, catechol, 3,3',5,5'-tetramethylbenzidine, p-tolidine, betanaphthol, pyrogallol, o-phenylenediamine, leuco malachite green, 3-amino ethylcarbazole, 7-amino antipyrine, and ABTS.

The two principal aspects of the invention may be employed separately or combined to improve FOBT. As indicated one is the use of a mixture of guaiac and ABTS as a chromagen in FOBT. The other is applying to the fecal specimen a developer or complete reagent solution that uses as the solvent system a solvent for iron protoporphyrins.

The chromagen mixture of the invention may be used in various FOBT formats that involve contacting a fecal specimen placed on a solid test matrix with a leuco dye and a hydroperoxide. In one format the matrix is impregnated with the mixture (i.e., the matrix carries the mixture in dry form) beforehand. In another the matrix is impregnated with one of the components of the mixture and the other is applied in solution to the matrix/specimen either combined with or separately from the hydroperoxide. For long-term stability, it is preferred to keep the chromagen mixture and hydroperoxide separate. For instance, they might be packaged in separate containers adapted for simultaneous dispensing, such as a double-barreled syringe with a common outlet nozzle. Another format is to employ the chromagen in solution as part of a complete reagent (when long-term stability is not involved). The complete reagent contains, in addition to the mixture and the solvent, a hydroperoxide and, optionally, other additives such as hemoprotein solubilizing agents, stabilizers, vegetable peroxidase inhibitors, iron chelators, and buffers. Such additives may, of course, be impregnated into the solid test matrix in the other test formats. Use of a complete reagent has the advantages of avoiding the need to impregnate the matrix beforehand, permitting new test geometries and use of untreated matrixes, and improving test performance.

In addition to use in FOBT, the chromagen mixture may be used advantageously in other common colorimetric tests for the presence of peroxidatively active molecules other than hemoproteins.

The chromagen mixture of guaiac and ABTS produces a color response that is greater than the sum of the responses that would be expected from the individual components. In addition to enhanced color intensity, the color is spread more evenly than the observed in current (e.g., HEMOCCULT® test) FOBT, is more stable (i.e., long-lasting), and is more reproducible. The weight ratio of guaiac to ABTS in the mixture will be in the range of 1:5 to 5:1, preferably approximately 1:1. When the chromagen mixture is formulated as a complete reagent with a peroxide and/or in a solvent for iron protoporphyrins such as DMSO, it is preferable to include a stabilizing amount of sodium sulfite in the formulation. Preferably the sodium sulfite is present in amounts in excess of that which saturates the solution. The concentration of the chromagen mixture in the solution will usually be in the range of 0.5 to 10% by weight.

Similarly, the application to the fecal specimen of a developer/complete reagent having a solvent system based on a solvent for iron protoporphyrins may be used in a variety of FOBT formats. When used as a developer, the solution will contain the hydroperoxide and be applied to the specimen on a leuco dye-impregnated matrix. When used in the form of a complete reagent, the solution will contain a leuco dye, preferably the multi-chromagen of the invention, together with the hydroperoxide and, optionally, other additives such as those described above.

The test matrixes used in the FOBT methods of the invention may be made from a variety of porous materials such as cellulosics (wood, paper), ceramic, glass fibers, natural or synthetic cloth fibers, felt, and sponge. Bibulous filter paper is commonly used and is preferred.

The solvents for iron protoporphyrins that may be used in the invention are aprotic solvents such as dimethylsulfoxide (DMSO), dimethyl formamide (DMF), and pyridine. DMSO is preferred. Cosolvents such as water, alkanols (e.g., methanol, ethanol, and other lower alkanols), pentane, ethylacetate, cyclohexane, and acetone may be included with the iron protoporphyrin solvent, but the proportion of principal solvent in the solvent compositions should be maintained above about 50% (v/v), preferably above about 75% (v/v).

The use of DMSO as the solvent in the developer/complete reagent provides several advantages. Firstly, it is an excellent solvent for hemoproteins, including iron protoporphyrins. Secondly, it converts heme and hemin dimers and aggregates into the monomeric forms. And, thirdly, it inactivates peroxidases that may be present in the fecal specimen and inhibits them from catalyzing the oxidation of the indicator and producing a false positive result.

Hemoprotein solubilizing agents that may be optionally included in the solution or test matrix include detergents and water-soluble polymers. Detergents that have suitable hydrophilic-hydrophobic balance to solubilize hemoproteins are suitable. Such detergents include the TRITON® detergents (polyoxyethylene alkylphenols), detergents from the series alkyltrimethylammonium bromides, like cetyl alkyltrimethylammonium bromide (CTAB) or p-toluene sulfonic acid salts of alkyltrimethylammonium bromide detergents, and $C_{10}$ to $C_{14}$ alkali metal salts of fatty acids or alkali metal alkyl sulfates. Examples of suitable detergents are sodium dodecyl sulfate (SDS), sodium dodecyl sulfonate, sodium decyl sulfate, sodium tetradecyl sulfate, sodium tridecyl sulfonate, sodium myristate, sodium caprate, sodium dodecyl N-sarcosinate, and sodium tetradecyl N-sacrosinate. The water-soluble polymers that may be used to solubilize hemoproteins include poly(ethylene oxide), poly(vinyl alcohol), poly(vinyl pyrrolidone), and poly(styrene sulfonate). These solubilizing agents not only solubilize hemoproteins, but are believed to convert peroxidatively inactive hemoprotein dimers or aggregates into peroxidatively active monomeric species.

Nitrogenous ligands that stabilize iron protoporphyrins and enhance their peroxidative activity may also be incorporated in the complete reagent or test matrix. Examples of such ligands are pyridine, histidine, caffeine, imidazole, and imidazole derivatives.

Other additives such as vegetable peroxidase inhibitors and iron chelators (e.g., ethylenediamine tetraacetic acid, citric acid, tartaric acid, nitrilotriacetic acid, diethylenetriamine-pentaacetic acid, N,N[1]-bishydroxyethyl glycine, ethyleneglycol bis(2- aminoethylether)tetraacetic acid, N-hydroxyethyl-ethylenediaminetetraacetic acid) may be incorporated into the test matrix to further reduce the likelihood of false test results. Buffers are added to maintain a suitable pH range for oxidizing the leuco dye. The particular buffer (pH range) will depend on the leuco dye that is used. The pH will usually be between about 3 and about 9. By way of example, guaiac oxidation is buffered at pH 6-7.5 (phosphate buffer), 3,3',5,5'-tetramethylbenzidine oxidation is buffered at a pH of about 4 (acetate buffer), and ABTS is buffered at a pH of about 9-9.5 (glycine buffer). For the chromagen mixture of the invention the pH should be in the range of 5 to 10.

Hydrogen peroxide or organic hydroperoxides such as cumene hydroperoxide, t-butyl hydroperoxide, diisopropylbenzene hydroperoxide, and 2,5-dimethylhexane hydroperoxide may be used in the developer or complete reagent. It is preferred to use an organic hydroperoxide since organic hydroperoxides are less likely to (a) produce false positive results in FOBT in which vegetable peroxidases are present in the fecal specimen and (b) destroy the peroxidase activity of the hemoprotein. The concentration of hydroperoxide in

4

the developer/complete reagent will usually be in the range of 0.05 to 10% by volume, more usually 0.5 to 5% by volume.

The FOBT materials and procedures of the invention and their advantages over previous materials and procedures are illustrated by the following examples. These examples are not intended to limit the invention in any manner.

Example 1

Tests were carried out to compare the specificity and sensitivity achieved in FOBT with a developer composition consisting of (5% (v/v) cumene hydroperoxide (CHP) in DMSO) versus that achieved with a conventional FOBT developer (3-6% (v/v) hydrogen peroxide in ethanol/water).

Serial dilutions of hemin (Hm, 0.1 mg/ml in 1mM ammonium hydroxide), hemoglobin (Hb, 1.0 mg/ml), and horseradish peroxidase (HRPO, 20 U/ml) in water were prepared. Two $\mu$l of these dilutions were applied to HEMOCCULT® slides and the spots were allowed to dry. Five $\mu$l of developer composition was applied to each spot. After one minute the presence or absence of blue color on the slide was noted ( + + + = intense color; + + = strong color; + = clearly visible color; ± = barely perceptible color; - = no detectable color). The results of these tests are shown in Table 1 below.

Table 1

| Dilution | Hm | | Hb | | HRPO | |
|---|---|---|---|---|---|---|
| | $H_2O_2$ | CHP | $H_2O_2$ | CHP | $H_2O_2$ | CHP |
| 0 | + + + | + + + | + + + | + + + | + + + | + + |
| 2 | + + + | + + + | + + + | + + + | + + + | + |
| 4 | + + + | + + + | + + + | + + + | + + + | ± |
| 8 | + + | + + | + + + | + + | + + + | - |
| 16 | ± | + | + | + + | + + + | - |
| 32 | - | ± | ± | + | + + | - |
| 64 | - | - | ± | ± | + + | - |
| 128 | - | - | - | ± | + | - |
| 256 | - | - | - | - | ± | - |
| 512 | - | - | - | - | ± | - |

As indicated by the results of Table 1, the DMSO-based developer was more sensitive than the conventional developer with hemoglobin and hemin and much less sensitive than the conventional developer with horseradish peroxidase. The invention developer thus improves the sensitivity and specificity of the test. Sensitivity can be improved by modifying the developer as needed.

Example 2

Hemin degradation tests were carried out using conventional FOBT developer and DMSO-based FOBT developer compositions of the present invention as follows.

Hemin was dissolved in a 1:10 (v/v) mixture of DMSO and water. Two $\mu$l of this solution was spotted onto filter paper and the paper was air dried. Spotted areas were then treated with 5 $\mu$l of one of the following developers: (a) CHP, 5%, in DMSO; (b) t-butyl hydroperoxide, 5%, in DMSO; (c) $H_2O_2$, 5% in water; (d) HEMOCCULT® developer ($H_2O_2$, 5%, in ethanol/water); (e) DMSO alone; (f) water alone; and (g) 75% ethanol, 25% water. The developer-treated areas were then air dried and 10 $\mu$l of guaiac solution (30 g/1200 ml isopropanol) was applied to each spot followed by air drying. Each spot was then treated with 15 $\mu$l HEMOCCULT® developer and color change was observed. Visible blue color was noted on each spot except those treated with (c) and (d). These results indicate that organic hydroperoxides in DMSO do not effect adversely the catalytic activity of hemin, whereas conventional FOBT developer does have an adverse effect on hemin activity in FOBT. Similar results were observed using native and denatured hemoglobin instead of hemin.

Example 3

Tests were conducted using developer compositions consisting of CHP at 5% in DMSO with various cosolvents. The procedure of Example 1 was used except that the highest clearly detectable dilution of catalyst is noted rather than color intensity. Conventional developer and CHP in other solvents were run for comparison. The results are reported in Table 2 below.

## Table 2

| | | Highest Detectable Dilution | | |
|---|---|---|---|---|
| Oxidant | Solvent | Hm | Hb | HRPO |
| Hydrogen Peroxide | ~75% ethanol | 5 | 5 | 10 |
| CHP | 100% DMSO | 6 | ND | 4 |
| CHP | 75% DMSO 25% Pentane | 5 | ND | 5 |
| CHP | 50% DMSO 50% Pentane | 5 | ND | 6 |
| CHP | 75% DMSO 25% ethyl acetate | 5 | ND | 5 |
| CHP | 75% DMSO 25% Cyclohexane | 5 | ND | 5 |
| CHP | 50% DMSO 50% Cyclohexane | 5 | ND | 5 |
| CHP | Dimethyl Formamide | 7 | 8 | 8 |
| CHP | Acetonitrile | 7 | ND | 9 |
| CHP | 90% DMSO 10% Acetone | 4 | 4 | 3 |
| CHP | 75% DMSO 25% Acetone | 4 | 5 | 3 |
| CHP | 50% DMSO 50% Acetone | 4 | 5 | 5 |
| CHP | 100% EtOH | 4 | 6 | 7 |
| CHP | 100% MeOH | 5 | 8 | 7 |

ND = not determined

The results of Table 2 show that CHP developer with DMSO at > 50% show reduced sensitivity to HRPO while retaining sensitivity for hemin. The best results occur using DMSO at 75% and above.

Example 3

Tests were carried out to assess the effect of the developer composition on the mobility of hemin spotted on filter paper. This is an indirect measure of the solubility of hemin in the developer.

Whatman filter paper was soaked with 10 $\mu$l of a 1 mg/ml hemin solution in 1 mM aqueous ammonium hydroxide. After drying, a small section of the hemin-treated paper was placed on a HEMOCCULT® slide. Ten $\mu$l of developer compositions comprising 5% CHP in DMSO/water solvent containing 100%, 95%, 90%, 80%, 75%, 50%, and 25% (v/v) DMSO were placed on the hemin spots and blue color formation was observed. At DMSO concentrations above about 75% blue color was noted in the entire slide area wetted by the developer. At lower DMSO concentrations color was observed only directly under the hemin-treated filter paper. A similar test with HEMOCCULT® developer produced no color.

Similar tests using hemoglobin instead of hemin gave similar results.

Example 4

The effect of hemoglobin denaturation on FOBT sensitivity using a DMSO-based developer composition of the invention and HEMOCCULT® developer was determined as follows.

Hemoglobin was denatured by treatment with (a) hydrochloric acid, (b) enzymes or (c) heat as follows:

6

(a) <u>Acid</u>

Dilute (0.1 M) hydrochloric acid was added to a solution of hemoglobin in water (5 mg/ml) until the pH of the solution was 1.0. The solution was incubated for 1 hr at 37°C and the pH adjusted to 7.4 with 0.1 N sodium hydroxide. The resulting material was homogenized to produce a finely dispersed suspension and incubated at 37°C for 48 hr.

(b) <u>Enzymes</u>

Solid trypsin (1 mg) and chymotrypsin (1 mg) were added to a solution of hemoglobin in water (5 mg/ml) and incubated for 48 hr at 37°C. The resulting mixture was homogenized to produce a smooth suspension.

(c) <u>Heat</u>

A solution of hemoglobin in water (5 mg/ml) was heated to 75°C for 10 min and incubated at 37°C for 48 hr. The resulting mixture was homogenized to produce a smooth suspension.

Whatman filter paper was spotted with 2 $\mu$l of an aqueous solution (5 mg/ml) of the denatured hemoglobins or native hemoglobin and allowed to dry. The spot of paper containing native or denatured hemoglobin was cut out from the filter paper sheet and place directly onto a HEMOCCULT® slide. The slides were then treated with developer compositions consisting of 5% CHP in DMSO/water at varying DMSO concentrations. Comparison slides were treated with HEMOCCULT® developer. Color intensities were observed as above. Table 3 reports the results of these tests. (+5 = extremely intense color; +1 = slight color; - = no color; ± = barely detectable; ND = not done.)

## <u>Table 3</u>

| | | Color Intensity | | |
|---|---|---|---|---|
| Percent DMSO | Acid Treated Hb | Enzyme Treated Hb | Heat Treated Hb | Native Hb |
| 100 | 2 | 3 | 5 | 5 |
| 95 | 2 | 3 | 5 | 5 |
| 90 | 1 | 3 | 4 | 4 |
| 85 | 1 | 2 | 4 | 4 |
| 75 | 1 | 2 | 2 | 2 |
| 50 | 1 | 1 | ± | 3 |
| 25 | ± | ± | ND | ± |
| HEMOCCULT® Developer | – | – | – | – |

The data reported in Table 3 indicate that the sensitivity of the FOBT using the DMSO-based developer is not affected by hemoglobin denaturation as much as FOBT using conventional developers. This is particularly significant since such denaturation is likely to occur to hemoglobin in the GI tract.

<u>Example 5</u>

This example illustrates the enhanced readability of the multi-chromagen FOBT reagent as regards color intensity and diffusiveness.

The following reagents were prepared:

A. HEMOCCULT® developer (6% hydrogen peroxide in alcohol);

B. 5% cumene hydroperoxide and 2% guaiac in 75% DMSO, 25% water; and

C. 5% cumene hydroperoxide, 2% guaiac, and 25 mg/ml ABTS in 75% DMSO, 25% water containing 100 mg solid sodium sulfite.

Standard HEMOCCULT® slides were smeared with untreated fecal specimens, or with specimens which had been spiked with hemin or hemoglobin (135 or 270 $\mu$g hemin/g feces, or with 5.7 mg hemoglobin/g

7

feces). After the smears had dried, the appropriate reagent was added, and the appearance of the test noted at 1 min. The results are reported in Table 4.

## Table 4

| Fecal Specimen | Hydrogen Peroxide | CHP + Guaiac | ABTS + CHP + Guaiac |
|---|---|---|---|
| 1. Untreated | negative | negative | negative |
| 2. Hemin spiked (135 μg/g) | negative | +1 (hollow center) | +5 (solid circle) |
| 3. Hemin spiked (270 μg/g) | negative | +3 (hollow center) | +10 (solid circle) |
| 4. Hemoglobin spiked | +1 | +3 | +10 (solid circle) |

negative = no color produced.
+1 = very faint blue color.
+10 = extremely intense blue color.

hollow center = blue ring with color at edge ofsolvent front, and no color in center.
solid circle = even blue color throughout the area covered by the solvent.

Example 6

This example shows the relative sensitivity and readability of FOBTs using the multi-chromagen of the invention and reagents containing only ABTS or guaiac.

The following chromagen compositions were used:

A. 2% guaiac and 5% cumene hydroperoxide in 75% DMSO, 25% water.

B. ABTS (10 mg/ml) and 5% cumene hydroperoxide in 75% DMSO, 25% water.

C. 1% guaiac, 5% cumene hydroperoxide, and ABTS (5 mg/ml) in 75% DMSO, 25% water.

A solution of hemin in DMSO (10 mg/ml) was diluted with DMSO in serial fashion 1:2 and the dilutions were spotted (2 μl) onto standard Whatman #1 filter paper. Five μl of reagent was added and color formation noted at 60 sec. The results of these tests are reported in Table 5.

8

Table 5

| Color Formation | | | |
|---|---|---|---|
| Hemin Concentration (mg/ml) | Reagent A | Reagent B | Reagent C |
| 1.00 | + | - | + |
| 0.50 | + | - | + |
| 0.25 | + | - | + |
| 0.13 | + | - | + |
| 0.07 | + | - | + |
| 0.04 | + | - | + |
| 0.02 | + | - | + |
| 0.01 | + /- | - | + |
| 0.005 | + /- | - | + /- |
| 0.0025 | - | - | + /- |

Color Characteristics:

Reagent A = Blue ring at solvent front, but hollow, colorless center.
Reagent B = No color formed on standard filter paper.
Reagent C = Very intense blue color formed; color evenly distributed throughout the entire solvent area.

Example 7

This example shows the chromatographic advantages of the multi-chromagen reagent relative to a single chromagen reagent.

The following reagents were used:

A. Reagent A - 2% guaiac and 5% cumene hydroperoxide in DMSO.

B. Reagent B - Multi-chromagen reagent (2% guaiac, 5% cumene hydroperoxide, and 25 mg/ml ABTS in DMSO over 100 mg solid sodium sulfite).

Two $\mu$l of a hemin suspension in water (1 mg/ml) was spotted onto standard Whatman #4 filter paper. After the water had evaporated, varying volumes of Reagent A or Reagent B were added, and the appearance of the blue color noted. Table 6 reports the results of these tests.

Table 6

| Appearance of Spot | | |
|---|---|---|
| Reagent Volume | REAGENT A | REAGENT B |
| 5 $\mu$l | very slightly disperse | full circle, intense color |
| 10 $\mu$l | slightly disperse, hollow center | full circle, intense color |
| 15 $\mu$l | disperse circle, hollow center | full circle, intense color |
| 20 $\mu$l | very disperse circle, hollow center | intense color, very slightly disperse center |

Color Characteristics:

full circle = Intense blue circle with color evenly distributed throughout the entire area covered by the solvent.
very slightly disperse circle = Blue circle, but small, faint, colorless area at center of circle.
slightly disperse = Blue circle, approximately 20% of central area colorless.
disperse circle = Blue ring, approximately 50% of central area colorless.
very disperse circle = blue ring with >80% of the central area colorless; color is primarily localized at the solvent front.

Example 8

This example illustrates enhanced color intensity and uniformity obtained using the multi-chromagen reagent.

The following reagents were used:

A. 2% guaiac and 5% cumene hydroperoxide in DMSO.

B. 2% guaiac and 5% cumene hydroperoxide and ABTS (25 mg/ml) in DMSO containing 100 mg solid sodium sulfite.

Two $\mu l$ hemin solution (0.05 mg/ml) in DMSO was spotted onto untreated Whatman #1 filter paper. Ten $\mu l$ reagent A or B was added and the appearance noted in 1-3 min. Each test was run in triplicate. Table 7 reports the results of the tests.

Table 7

| REAGENT A | | | REAGENT B | |
|---|---|---|---|---|
| Intensity | | Appearance | Intensity | Appearance |
| 1. | +3 | Blue ring with hollow center | +10 | Solid blue circle intensely blue |
| 2. | +3 | Blue ring with hollow center | +10 | Solid blue circle intensely blue |
| 3. | +3 | Blue ring with hollow center | +10 | Solid blue circle intensely blue |

Color Characteristics:

| | |
|---|---|
| Blue ring with hollow center = | Blue color at edge of solvent front, but no color in center. |
| Solid blue circle = | Even, blue color throughout the area covered by the solvent. |
| +1 = | very faint blue |
| +10 = | extremely intense blue |

Example 9

This example provides a comparison of color intensity, stability and chromatography in tests using the HEMOCCULT® reagents and the multiple-chromagen of the invention, with hemin as catalyst.

The following reagents were used:

A. HEMOCCULT® developer (6% hydrogen peroxide in alcohol).

B. Multi-chromagen reagent (1% guaiac, 5% cumene hydroperoxide, and ABTS (25 mg/ml) in DMSO).

Two $\mu l$ of a suspension of hemin in water (0.25 mg/ml) was added to standard HEMOCCULT® slides, and the spots allowed to dry. Five $\mu l$ of each reagent was added to the spots, and color formation noted at 30 sec, 60 sec, 3 min, and 5 min. Table 8 reports the results of these tests.

Table 8

| COLOR FORMATION | | | | |
|---|---|---|---|---|
| HEMOCCULT DEVELOPER | | | MULTIPLE-CHROMAGEN REAGENT | |
| Time | Intensity | Appearance | Intensity | Appearance |
| 30 sec | +2 | diffuse, uneven color | +8 | intense full circle color |
| 60 sec | +1 | diffuse, uneven color | +10 | intense full circle color |
| 3 min | neg | no color | +7 | intense full circle color |
| 5 min | neg | no color | +7 | intense full circle color |

Color Characteristics:

| | |
|---|---|
| Reagent A = | Weak, diffuse color with streaking. |
| Reagent B = | Extremely intense blue color. Color is evenly distributed throughout the area covered |

by the solvent.

Example 10

This example reports the sensitivity of complete multiple-chromagen FOBT to plant peroxidases relative to other reagents.

The following reagents were used:

A. Horseradish Peroxidase, 1,000 Units/ml.

B. HEMOCCULT® developer (6% hydrogen peroxide in alcohol).

C. 5% cumene hydroperoxide in DMSO.

D. 5% cumene hydroperoxide in DMSO containing 2% guaiac.

E. 5% cumene hydroperoxide in DMSO containing 2% guaiac and ABTS (25 mg/ml) stored over solid sodium sulfite.

The horseradish peroxidase was diluted in serial fashion with 1:2 with distilled water. Two $\mu$l of the dilutions were spotted in quadruplicate on standard HEMOCCULT® slides. The spots were allowed to dry, and 5 $\mu$l of the appropriate reagent added. Color was determined at 60 sec. The results of these tests are reported in Table 9.

Table 9

| COLOR FORMATION | | | | |
|---|---|---|---|---|
| (Units/ml) | REAGENT B | REAGENT C | REAGENT D | REAGENT E |
| 2.00 | + | + | + | + |
| 1.00 | + | + | + | + |
| 0.50 | + | + | + | + |
| 0.25 | + | + | + | - |
| 0.13 | + | + /- | + | - |
| 0.07 | + | - | + | - |
| 0.04 | + | - | + /- | - |
| 0.02 | + | - | + /- | - |
| 0.01 | + | - | - | - |
| 0.005 | + | - | - | - |
| 0.0025 | + /- | - | - | - |
| 0.0013 | + /- | - | - | - |
| 0.0007 | + /- | - | - | - |
| 0.0004 | + /- | - | - | - |
| 0.0002 | - | - | - | - |

Example 11

This example shows the effect of sulfite on the multiple-chromagen reagent stability.

Multiple component reagent containing 5% cumene hydroperoxide and 100 mg/ml ABTS in DMSO was made by adding guaiac or solid sodium sulfite as indicated in Table 10 below. Sodium sulfite was present either as excess solid, or as a saturated solution in DMSO from which all crystals were removed by filtration.

Procedure:

Each reagent was tested for its ability to detect 2 $\mu$l of a hemin solution (1 mg/ml) in DMSO on standard Whatman filter paper #1. Ten $\mu$l of reagent were used. The results are reported in Table 10.

Table 10

| REAGENT COMPOSITION | | | COLOR FORMATION | |
|---|---|---|---|---|
| ABTS (%) | Guaiac (%) | Sulfite Added | Day One | Day 120 |
| 1.0 | 2.0 | none | + | - |
| 1.0 | 2.0 | saturated (no excess solid) | + | - |
| 1.0 | 2.0 | excess solid | + | + |
| 1.0 | none | none | +/- | - |
| 1.0 | none | excess solid | +/- | - |

**Claims**

1.  A fecal occult blood test method comprising contacting a fecal specimen on a solid test matrix with a leuco dye and a hydroperoxide characterized in that the hydroperoxide or the hydroperoxide and the leuco dye are in solution in a solvent comprising at least 50% by volume of a solvent for iron protoporphyrins whereby the iron protoporphyrins in the specimen are dissolved.

2.  The method of claim 1 further characterized in that the solvent for iron protoporphyrins is dimethyl sulfoxide.

3.  The method of claim 1 or 2 further characterized in that the leuco dye comprises a mixture of guaiac and 2,2'-azino-di-(ethylbenzyl)thiazoline sulfonic acid (ABTS).

4.  The method of claim 3 further characterized in that the weight ratio of guaiac to ABTS is in the range of 1:5 to 5:1.

5.  The method of any one of claims 1 to 4 further characterized in that the solution contains a stabilizing amount of sodium sulfite.

6.  A fecal occult blood test method comprising contacting a fecal specimen on a solid test matrix with a leuco dye and a hydroperoxide characterized in that the leuco dye comprises a mixture of guaiac and ABTS.

European
Patent Office

EUROPEAN SEARCH
REPORT

Application Number

EP 91 11 8723

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-2 042 828   (BOEHRINGER MANNHEIM GMBH) <br> * the whole document * * <br> – – – | 1-6 | G 01 N 33/72 // <br> C 12 Q 1/28 |
| A | US-A-4 071 317   (C. T. W. LAM) <br> * claims * * <br> – – – | 1-6 | |
| A | FR-A-2 256 745   (SHIONOGI & CO., LTD.) <br> * examples 1-8 * * <br> – – – | 1-6 | |
| A | US-A-3 627 698   (H.- G. REY) <br> – – – | | |
| A | US-A-4 005 984   (R. F. ALSOP.) <br> – – – – – | | |

| TECHNICAL FIELDS SEARCHED (Int. Cl.5) |
|---|
| G 01 N <br> C 12 Q |

The present search report has been drawn up for all claims

| Place of search | Date of completion of search | Examiner |
|---|---|---|
| The Hague | 08 January 92 | GRIFFITH G. |

CATEGORY OF CITED DOCUMENTS

X : particularly relevant if taken alone
Y : particularly relevant if combined with another
    document of the same catagory
A : technological background
O : non-written disclosure
P : intermediate document
T : theory or principle underlying the invention

E : earlier patent document, but published on, or after
    the filing date
D : document cited in the application
L : document cited for other reasons

&: member of the same patent family, corresponding
    document